# EUROPEAN PATENT APPLICATION

(11) **EP 3 514 542 A1**
(43) Date of publication of application: **24.07.2019**
(21) Application number: 18153096.5
(22) Date of filing: 23.01.2018
(51) Int. Cl.: G01N 33/543, G01N 27/327

(54) **IMPEDANCE CHIP DETECTION SYSTEM FOR BIOLOGICAL TESTING**

(71) Applicant: Southern Taiwan University of Science and Technology, Tainan City 71005 (TW)
(72) Inventor: CHUANG, CHENG HSIN, TAINAN CITY (TW); DU, YI CHUN, TAINAN CITY (TW); ZHU, PEI YU, TAINAN CITY (TW)
(74) Representative: Lang, Christian

(57) **Abstract**

An impedance chip detection system for biological testing includes a sensor and a portable detector. The sensor includes an absorbing layer, a sensing layer, and a transmitting layer, and the detector includes a detection slot, a control module provided for an impedance measurement and calculation of a specimen, and a display module for displaying a detection result of the specimen, so that after the sensor is plugged into the detection slot, the sensor performs an impedance measurement and calculation of the specimen and the detector displays the detection result.

## Description

### FIELD OF THE INVENTION

The present invention relates to an impedance chip detection system for biological testing, and more particularly to the system using a detector with a portable circuit module to complete a biological testing after a specimen is sensed by a sensor.

### BACKGROUND OF THE INVENTION

### 1. Description of the Related Art

According to the market analysis survey of the Total Biosensor Market 2016, biological detectors used for home care will be the trend of biomedical detections in the future and it is estimated that over 65% of the users focus on home diagnosis, so that patients can have a simple, convenient, and low-cost instrument for the testing functions.

Since paper has the features of low cost, and high productivity and versatility, a sensor made of paper is applicable for testing with better flexibility, portability, disposability and simplicity. The portability, convenience, and immediacy are the main factors for paper sensors to gradually become the future trend of biosensors. Therefore, P.R.C. Pat. Publication No. 103041876 discloses a "Preparation of three-dimensional microfluidic paper chip and application of the three-dimensional microfluidic paper chip for a field electrochemical detection", wherein a hydrophobic pattern is printed on three filter papers by batch processing, and a working electrode, a reference electrode and a counter electrode are printed on the filter papers by matrix screen printing by batch processing after a molten wax molding, and horseradish peroxidase and oxidase are printed on a surface of the filter paper without the working electrode by batch processing, and then the filter papers are adhered by a two-sided adhesive tape after the paper chip is cut, so as to form the electrochemical three-dimensional microfluidic paper chip. However, the aforementioned paper sensor must be connected to an electrochemical workstation to read testing data, in order to obtain the intensity of the reacted current and complete the analysis and testing of biomaterials. Although the sensor can improve the convenience of the testing operation, the efficiency of the subsequent numerical measurement analysis cannot be improved. Obviously, the conventional paper sensor requires improvements.

### 2. Summary of the Invention

In view of the aforementioned drawback of the conventional paper sensor that cannot improve the efficiency of the subsequent numerical measurement analysis, the inventor of the present invention provides an impedance chip detection system for biological testing.

Therefore, it is a primary objective of the present invention to provide a system with a portable circuit detector for reading detected data.

To achieve the aforementioned and other objectives, the present invention provides an impedance chip detection system for biological testing, and the system comprises:
a sensor, applied for biological testing, and comprising: an absorbing layer, being an airlaid paper, for absorbing excessive specimen; a sensing layer, combined with the absorbing layer, and including a substrate with an electrode pattern, an antibody nanoparticle modified and disposed on a surface of the electrode pattern, and an adhesive combined with the top of the substrate, and solidified to form a hydrophobic area; and a transmitting layer, combined with the sensing layer by a hydrophilic glue, and including a filter paper, an adhesive combined with the top of the filter paper and solidified to form a hydrophobic area, and a flow channel for transporting a specimen; and

a detector, being a portable impedance chip detector, for detecting a specimen transported from the detector, and including: a housing, having a detection slot for plugging the sensor; a control module, for performing an impedance measurement and calculation of the specimen, and including a gain optimization module for automatically detecting plurality of detection points, and an impedance demodulation module for selecting the best detection point, and confirming whether the impedance calculation exceeds an error range after the impedance calculation is completed; and a display module, combined with the housing, and electrically coupled to the control module, for displaying a detection result of the specimen or a warning reminder.

According to the description above, the sensor can drive a detection electrode and a reference electrode to react at the same time through the transmitting layer and the sensing layer, and then the sensor is plugged into the detection slot of the detector and provided for an impedance measurement and calculation of a specimen, and the detector displays the detection result of the specimen or a warning reminder to improve the convenience and efficiency of a biomedical detection.

### BRIEF DESCRIPTION OF THE DRAWINGS

FIG. 1 is a perspective view of the present invention;
FIG. 2 is an exploded view of a sensor of the present invention;
FIGS. 3-A and 3-B are schematic views showing the manufacture of a detector of the present invention;
FIG. 4 is an exploded view of a sensor of the present invention;
FIG. 5 is a block diagram showing the circuit of a detector of the present invention;
FIGS. 6 and 7 are schematic views of the present invention performing a biological testing;
FIG. 8 is a flow chart of a biological testing of the present invention;
FIG. 9 is a graph showing a detection result of an experiment performed by a sensor of the present invention; and
FIG. 10 is a graph showing the comparison of the detection results obtained by the detector of the present invention sensor and a commercial measuring machine.

### DESCRIPTION OF THE PREFERRED EMBODIMENTS

The above and other objects, features and advantages of this disclosure will become apparent from the following detailed description taken with the accompanying drawings.

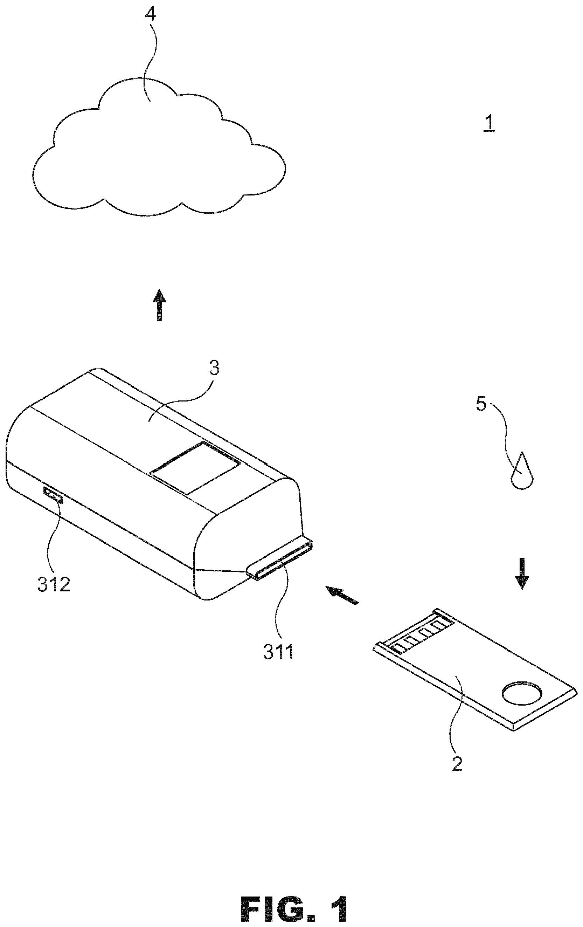

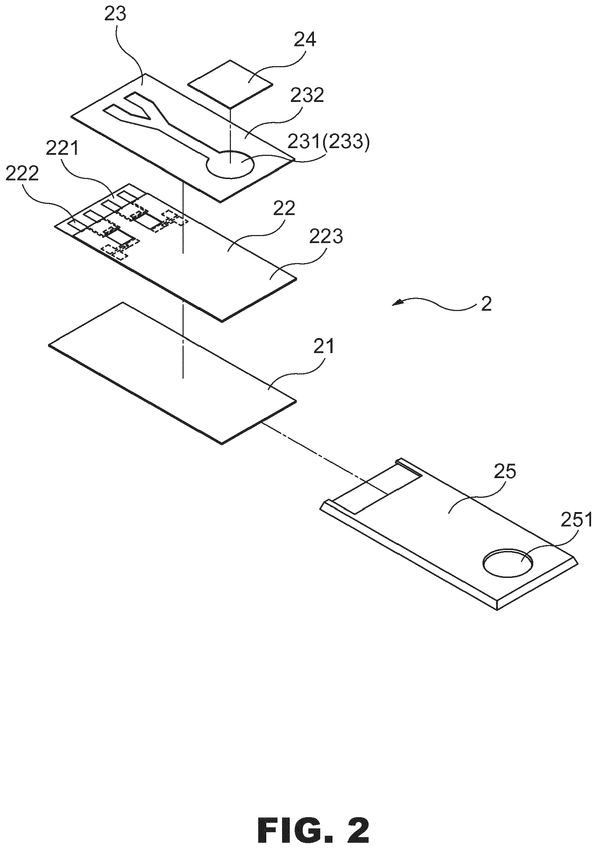

With reference to FIG. 1 for a system 1 of the present invention, the system 1 comprises a sensor 2, a detector 3 and a cloud system 4, wherein the sensor 2 is applied for biological testing and comprises an absorbing layer 21, a sensing layer 22 and a transmitting layer 23 (as shown FIG. 2), and the absorbing layer 21 is an airlaid paper, for absorbing excessive specimen; the sensing layer 22 is combined with the absorbing layer 21 and comprises a substrate 221 having an electrode pattern 222, and an antibody nanoparticle 224 (not shown in the figure) modified and disposed on a surface of the electrode pattern 222, an adhesive 223 combined with the top of the substrate 221 and solidified to form a hydrophobic area, wherein the substrate 221 of a preferred embodiment of the present invention is a paper substrate, a PET film, or a glass substrate; the transmitting layer 23 is combined with the sensing layer 22 by a hydrophilic glue and comprises a filter paper 231, an adhesive 232 combined with the top of the filter paper 231 and solidified to form a hydrophobic area, and a flow channel 233 for transporting a specimen. In a preferred embodiment of the present invention, the flow channel 233 includes a micro pump for driving the flow of the specimen.

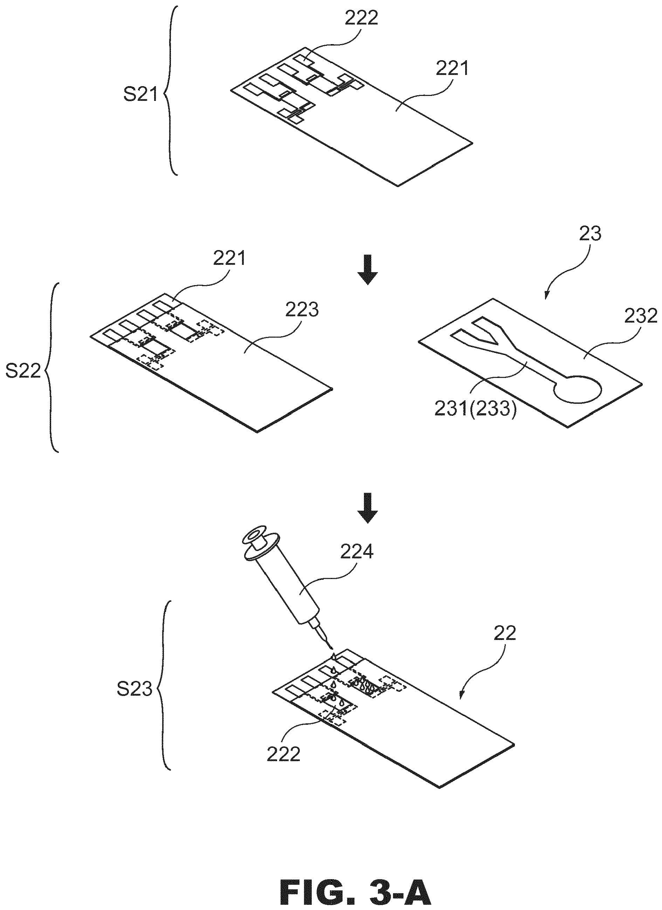

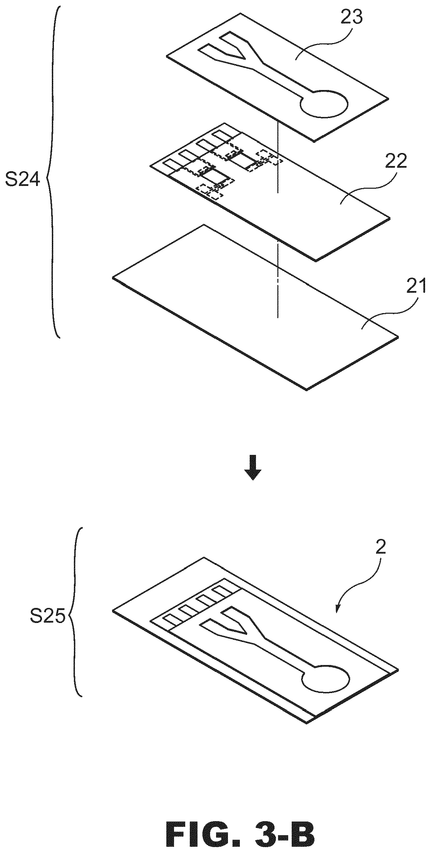

In an embodiment, a paper substrate sensor 2 is used, wherein the manufacturing process of the paper substrate sensor 2 (as shown in FIGS. 3-A and 3-B) comprises the following steps.
S21: Select a filter paper with a plurality of holes of an appropriate size to print a paper substrate 221, and a screen printing method or a gravure mold roll is used to print an electrically conductive solution onto the paper substrate 221 to form an electrode pattern 222 of a specific shape. Preferably, the electrically conductive solution is a precious metal solution, particularly a silver glue solution. The electrode pattern 222 is an interdigitated electrode with both left and right sides arranged in the shape of a comb.
S22: Spray an appropriate amount of ultraviolet adhesive 223 onto the paper substrate 221, and penetrate the adhesive 223 through the depth of the paper, and projecting a UV lamp to solidify the adhesive, so as to shelter a specific area and form a hydrophobic area on the paper substrate 221. Spray an appropriate amount of ultraviolet adhesive 232 onto another filter paper 231, and similarly use an UV lamp to solidify the adhesive, so that the filter paper 231 has a hydrophobic area. Form a flow channel 233 at a part of the filter paper 231 without having the sprayed adhesive, so as to form a transmitting layer 23.
S23: Modify the surface of the electrode pattern 222 and combine an antibody nanoparticle 224 of the testing molecules to form a sensing layer 22.
S24: Use a hydrophilic glue to attach the sensing layer 22 and the transmitting layer 23 onto an absorbing layer 21 sequentially, so that the absorbing layer 21 can absorb any excessive specimen, and a paper substrate sensor 2 is formed.
S25: Package the sensor 2 into a housing 25 having a window 251, and install filter layer 24 (see FIG. 2) between the housing 25 and the sensor 2.

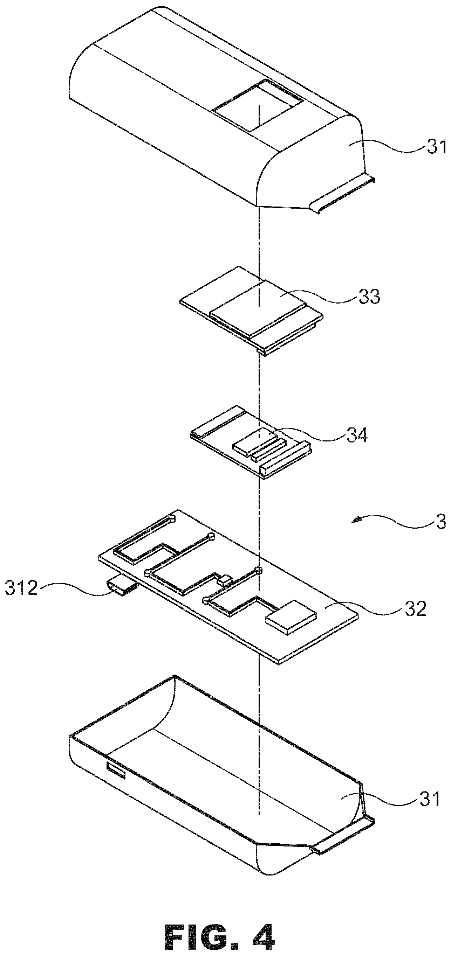

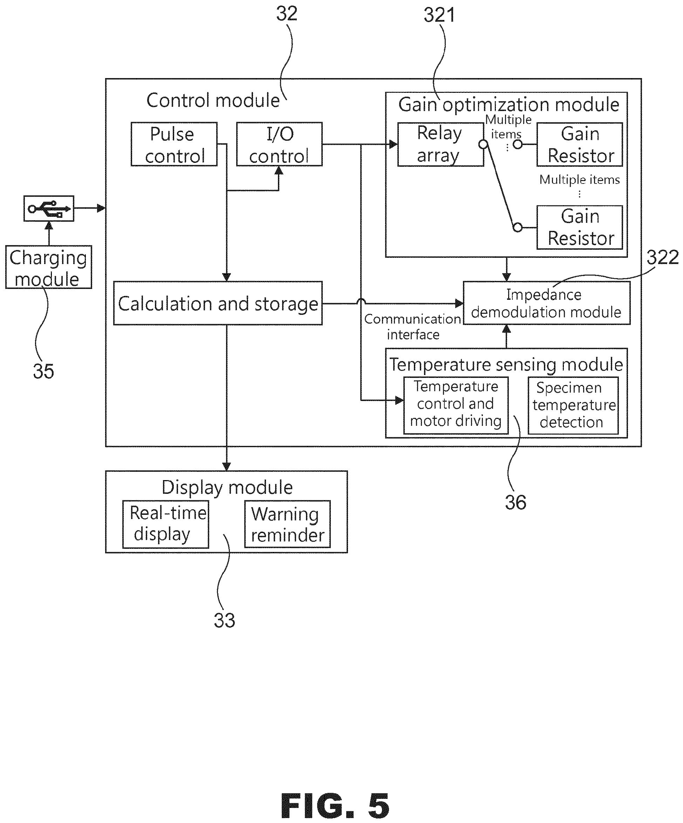

With reference to FIGS. 4 and 5 for a detector 3 of the present invention, the detector 3 is a portable impedance chip detector 3 for detecting the specimen transported from the sensor 2 and comprises a housing 31, a control module 32, a display module 33, a wireless transmission device 34, a charging module 35 and a temperature sensing module 36, wherein the housing 31 has a detection slot 311 (See FIG. 1) provided for plugging the sensor 2; the control module 32 is used for an impedance measurement and calculation of the specimen 5 on the sensor 2, and comprises a gain optimization module 321 for automatically detecting a plurality of detection points and an impedance demodulation module 322 for selecting the best detection point, and confirming whether or not the result exceeds an error range after the impedance calculation is completed. In an embodiment of the present invention, the circuit of the control module 32 is a Field-Programmable Gate Array (FPGA) chip. The display module 33 comprises an Organic Light-Emitting Diode (OLED) combined with the housing 31 and electrically coupled to the control module 32 for displaying a detection result of the specimen 5 or a warning reminder. The wireless transmission device 34 is a Bluetooth device provided for transmitting the detection result of the sensor 2 to a cloud system 4. The charging module 35 has a USB slot 312 formed on the housing 31 for connecting an external electrical power, so as to supply or store electrical power required for the detector 3 through the slot 312. The temperature sensing module 36 is provided for sensing and determining whether or not the temperature of the specimen 5 falls within a predetermined range, and increasing the temperature when the temperature of the specimen 5 has not reached the predetermined range.

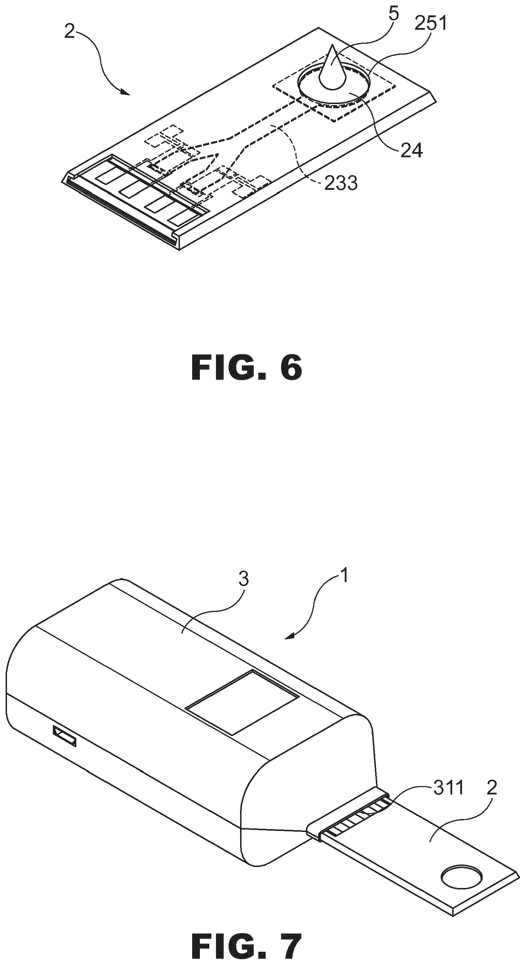

With reference to FIGS. 6 and 7, the prevalence of chronic kidney disease in different countries of the world has a trend of rising year by year, and approximately 90% of the patients in Taiwan have no idea about their illness and delay treatment. Therefore, a research experiment applies the design of the system 1 of the present invention to detect and measure the protein content in urine, wherein a 1-micron polyaniline micro probe (PANI MPs) coats an electrical polyaniline (PANDB) material on a particle surface of aluminum oxide (Al₂O₃) by redox, and modifies the particle surface by a silane functional group provided for the bonding of the antibody to form the probe and serves as a biometric component, and a probe of an antibody without bonds is used as control, and the probes of the antibody having bonds and the antibody without bonds are modified on surfaces of the detection area and the reference area respectively.

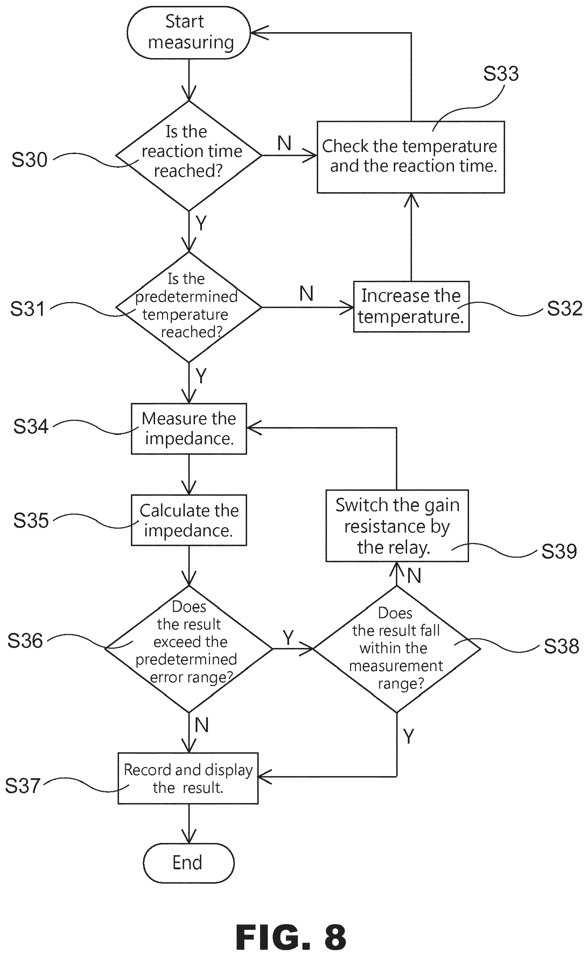

In this experiment, after the detector 3 is turned on, warmed up, and self-corrected (See FIG. 8), the paper substrate sensor 2 is plugged into the detection slot 311 of the detector 3, and deionized water is dropped onto the window 251 of the sensor 2 for the measurement of the initial value (Z0) of the chip, and then a protein solution (which is the specimen 5) is dropped onto the paper substrate sensor 2. With the buffered by the filter layer 24, a micro pump of the flow channel 233 achieves the best flow rate 389µL/min, and the back pressure 6cm-H₂O by inputting a voltage of 5V and a frequency of 40Hz, so as to drive the specimen 5 to flow on the surface of the biochip and is guided to the detection area and the reference area.
S30: Produce an immunoassay reaction between the biological antibody and the protein for 5 minutes.
S31: Sense the temperature of the specimen 5 by the temperature sensing module 36. In this embodiment, the preferred temperature predetermined range is 26°C∼30°C.
S32: Increase the temperature of the specimen 5 by driving the temperature control of the temperature sensing module 36 and the motor, if the temperature of the specimen 5 has not reached the predetermined range.
S33: Check the temperature and the reaction time again after the temperature is increased.
S34: Perform an impedance measurement after the dropped deionized water is rinsed.
S35: Calculate the impedance. Since the antigen in the reacted protein solution will produce a self-gratification with the antibody, therefore the reaction result of the specimen 5 can be obtained by deriving the impedance variation rate.
S3 6: Determine whether or not the derived result exceeds the predetermined range.
S37: Record and display the detection result from the detector 3, if the derived result does not exceed the predetermined range.
S38: Check whether or not the derived result falls within a measurement range, if the derived result exceeds the predetermined range.
S39: Use a relay array of the gain optimization module 321 of the control module 32 to switch the gain resistance, and use the impedance demodulation module 322 to select and return the best detection point, and then return to the step S34 to perform the impedance measurement again.

At present, the range of detecting the disease corresponding to the protein can be divided into the following five stages:
Chronic kidney disease stage 1: (Abnormal urine, but normal kidney function), Albumin content <30mg/24hr.
Chronic kidney disease stage 2: (Mild chronic kidney disease), Albumin content <30mg/24hr.
Chronic kidney disease stage 3: (Moderate chronic kidney disease), Albumin content 30∼299mg/24hr.
Chronic kidney disease stage 4: (Major chronic kidney disease), Albumin content>300mg/24hr.
Chronic kidney disease stage 5: (End-stage kidney disease), Albumin content >300mg/24hr.

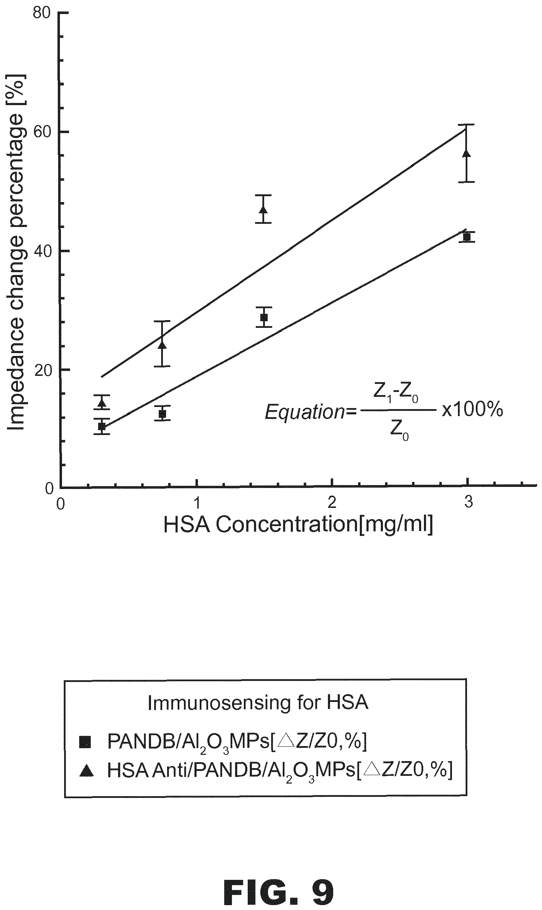

With reference to FIG. 9 for the result of this experiment, the detection variation rate of the sensor 2 is approximately 14 times, and has reached the lowest detection limit of the Albumin concentration (0.3mg/ml, as shown in Table 1) which is the same as the limit of the conventional commercial test paper. Therefore, we can derive that the sensor 2 of the present invention can achieve the distinction of the chronic kidney disease stage 4.

**Table 1**

| HSA concentration | 0.3 mg/ml | 0.75 mg/ml | 1.5 mg/ml | 3 mg/ml |
|---|---|---|---|---|
| PANDB with AHSA | 14.32±1.04% | 24.08±3.78% | 46.68±2.34% | 55.95±4.85% |
| PANDB without AHSA | 10.31±1.37% | 12.5±1.13% | 28.50±1.63% | 41.87±0.75% |
| Variation | 4.01±0.33% | 11.58±2.65% | 18.18±0.71% | 14.08±4.1% |
| Ratio | 1.3362 | 1.6376 | 1.9264 | 1.3887 |

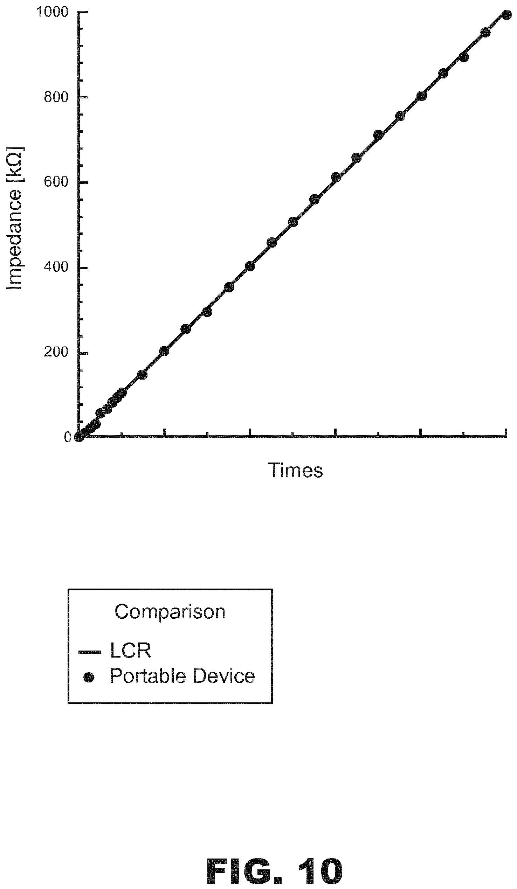

In addition, the detector 3 of the present invention measures the frequency 10KHz by a comparison range 1K∼1M(Ω), and compares the result by a commercial impedance analyzer (LCR Meter) (as shown in FIG. 10), and the comparison result shows that the error value of the detector 3 of the present invention is controlled within a range of 1∼2%, so that there is no significant difference between the result with the numerical value of the commercial impedance analyzer, and the invention can be used to replace the conventional impedance analyzer having a large volume.

Therefore, the system of the present invention has the following effects:
1. The sensor of the present invention can be operated together with a biological immune response mechanism for the application in various different testing areas, and the sensor of the invention with the advantages of multiple functions, fast, high sensitivity, and low cost can be used in various different testing areas such as clinical and chemical analyses, disease detection, food safety, pesticide residue, flu virus or drug screening, etc.
2. The portable detector of the present invention comes with a small volume, so that it can be used to replace the conventional impedance analyzer with a large volume and allow patients to use a low-cost equipment to achieve the detection function at home and improve the convenience of home care.
3. The detector of the present invention can be operated together with the sensor for detecting a specimen and uploading the detection result to a cloud system through a wireless transmission device, and the detection result is provided to medical care workers or medical institutions as a reference or a basis for tracking and medical treatment.

While the invention has been described by means of specific embodiments, numerous modifications and variations could be made thereto by those skilled in the art without departing from the scope and spirit of the invention set forth in the claims.

In summation of the above description, the present invention herein enhances the performance over the conventional structure and further complies with the patent application requirements and is submitted to the Patent and Trademark Office for review and granting of the commensurate patent rights.

## Claims

1. An impedance chip detection system for biological testing, comprising:
a sensor, applied for biological testing, and comprising:
an absorbing layer, being an airlaid paper, for absorbing excessive specimen;
a sensing layer, combined with the absorbing layer, and including a substrate with an electrode pattern, an antibody nanoparticle modified and disposed on a surface of the electrode pattern, and an adhesive combined with the top of the substrate, and solidified to form a hydrophobic area; and
a transmitting layer, combined with the sensing layer by a hydrophilic glue, and including a filter paper, an adhesive combined with the top of the filter paper and solidified to form a hydrophobic area, and a flow channel for transporting a specimen; and
a detector, being a portable impedance chip detector, for detecting a specimen transported from the detector, and including:
a housing, having a detection slot for plugging the sensor;
a control module, for performing an impedance measurement and calculation of the specimen, and including a gain optimization module for automatically detecting plurality of detection points, and an impedance demodulation module for selecting the best detection point, and confirming whether the impedance calculation exceeds an error range after the impedance calculation is completed; and
a display module, combined with the housing, and electrically coupled to the control module, for displaying a detection result of the specimen or a warning reminder.

2. The impedance chip detection system for biological testing according to claim 1, wherein the substrate of the sensor is a paper substrate, a PET film, or a glass substrate.

3. The impedance chip detection system for biological testing according to claim 1, wherein the electrode pattern of the substrate is an interdigitated electrode.

4. The impedance chip detection system for biological testing according to claim 1, wherein the flow channel further comprises a micro pump for driving the flow of the specimen.

5. The impedance chip detection system for biological testing according to claim 1, wherein the detector further comprises a wireless transmission device for transmitting the detection result to a cloud system.

6. The impedance chip detection system for biological testing according to claim 5, wherein the wireless transmission device is a Bluetooth device.

7. The impedance chip detection system for biological testing according to claim 1, wherein the detector further comprises a charging module, an external electrical power slot formed on the housing and provided for connection, so that the slot can provide or store electric power required for the detector.

8. The impedance chip detection system for biological testing according to claim 1, wherein the circuit of the control module of the detector is a Field-Programmable Gate Array (FPGA) chip.

9. The impedance chip detection system for biological testing according to claim 1, wherein the detector further comprises a temperature sensing module for detecting and determining whether or not the temperature of the specimen falls within a predetermined range, and increasing the temperature when the sensed temperature has not reached the predetermined range.

10. The impedance chip detection system for biological testing according to claim 9, wherein the predetermined range of temperature of the temperature sensing module is 26°C∼30°C.
